Europäisches Patentamt

European Patent Office

Office européen des brevets

(19)

(11) **EP 1 298 217 A1**

(12)

# EUROPEAN PATENT APPLICATION
published in accordance with Art. 158(3) EPC

(43) Date of publication:
**02.04.2003 Bulletin 2003/14**

(21) Application number: **01949913.6**

(22) Date of filing: **04.07.2001**

(51) Int Cl.⁷: **C12P 21/00**, C12N 15/10,
C07K 14/00
// C12P21:00, C12R1:91

(86) International application number:
**PCT/JP01/05812**

(87) International publication number:
**WO 02/002793 (10.01.2002 Gazette 2002/02)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **05.07.2000 JP 2000204327**

(71) Applicants:
• **Mitsubishi Pharma Corporation
Osaka-shi, Osaka 541-0046 (JP)**
• **WELFIDE CORPORATION
Osaka 541-0046 (JP)**

(72) Inventors:
• **SUGA, Kenichi,
c/o GRADUATE SCHOOL OF ENGINEERING
Suita-shi, Osaka 565-0871 (JP)**
• **OMASA, Takeshi,
c/o GRADUATE SCHOOL OF ENGINEERING
Suita-shi, Osaka 565-0871 (JP)**

• **KISHIMOTO, Michimasa,
c/o GRADUATE SCHOOL OF ENGIN
Suita-shi, Osaka565-0871 (JP)**
• **KATAKURA, Yoshio,
c/o GRADUATE SCHOOL OF ENGIN
Suita-shi, Osaka 565-0871 (JP)**
• **OHDA, Toyoo,
c/o Mitsubishi Pharma Corporation
Chuo-ku, Tokyo 103-8405 (JP)**
• **MIKI, Hideo,c/o Mitsubishi Pharma Corporation
Chuo-ku, Tokyo 103-8405 (JP)**
• **KOBAYASHI, Kaoru,
Mitsubishi Pharma Corporation
Chuo-ku, Tokyo 103-8405 (JP)**

(74) Representative: **Krauss, Jan
Forrester & Boehmert,
Pettenkoferstrasse 20-22
80336 München (DE)**

(54) **PROCESS FOR PRODUCING GLYCOPROTEIN**

(57)    A means of controlling sugar chain-modification and sugar chain structure in the production of a glycoprotein with the use of gene recombination techniques. A process for producing a glycoprotein by culturing a gene recombinant host in a medium characterized in that the relative sugar consumption speed is employed as an indication and changed to thereby modify the sugar chain structure attached to the protein.

**EP 1 298 217 A1**

**Description**

TECHNICAL FIELD

The present invention relates to a method for producing a glycoprotein.

BACKGROUND ART

Recently attention has been paid to the production of genetically recombinant, useful, and physiologically active substances. When a glycoprotein is produced among these, a cell capable of mainly modifying a sugar chain such as a eukaryotic cell (e.g., mammalian cell) must be used as a host.
The modification of the sugar chain is not as strictly controlled on a DNA level as seen in the production of a protein. The modification is carried out by a series of enzymatic reactions consisting of up to about 20 steps, so that it is known that the process of the sugar chain modification is affected by factors such as protein structure, host cell lines, and culturing condition for cells (*Biotechnology*, vol. 8, pp. 421-428, 1990; ibid., vol. 13, pp. 592-596, 1995; *Biochemistry*, vol. 28, pp. 7644-7662, 1989).
Therefore, when a glycoprotein is produced in genetically engineered cell line, natural-type and recombinant-type sugar chains can have different structures, and recombinant-type sugar chains can have macro- and micro-heterogeneity of carbohydrate structures in many cases *(J. Biol. Chem.,* pp. 21153-21159, 1989; *Arch. Biochem. Biophys.,* vol. 203, pp. 458-465, 1980).
It has been elucidated that the sugar chain of a glycoprotein affects the stabilization of the three-dimensional structure of a protein, the defense against the degradation of a protein, the promotion of the excretion of a protein, the physiological activity of the glycoprotein, and so on (*Mol. Cell Biochem.*, vol. 72, pp. 3-20, 1986; *Glycobiology*, vol. 1, 115-130, 1991; *Tampakushitsu Kakusan Kouso* vol. 37, pp. 1713-1746, 1992; *Thromb. Haemostas.,* vol. 60, pp. 255-261, 1988; *FASEBJ*, vol. 9, pp. 115-119, 1995).
In case a recombinant glycoprotein is used for therapeutic use, a sugar chain structure different from a natural-type one can cause an immunological reaction, so that it is desirable that a recombinant-type one has a sugar chain structure common to the natural-type one. Thus, controlling the structure of the sugar chain is a big problem in the production of a recombinant glycoprotein.
A few methods have been reported concerning the control of the structure of the sugar chain such as method for modifying composition of carbohydrate or molecular weight of a glycoprotein produced by changing the composition or concentration of a sugar in the medium (Japanese Patent Application Laid-Open (*kokai*) No. Hei 6-292592) and method for suppressing the transfer of galactose by adding glucosamine or N-acetylglucosamine into a medium (Japanese Patent Application Laid-Open (*kokai*) No. Hei 11-127890). Both of these are related with the production of an antibody using hybridoma cells.
The object of the present invention is to provide means for controlling the "modification and structure change" of a sugar chain in the production of a glycoprotein using the genetically recombinant technique.

DISCLOSURE OF THE INVENTION

As a result of study in view of the above circumstances, the inventors have found that there is certain relevance between the sugar specific consumption rate and the sugar chain modification and conceived the present invention. That is, the present invention is related to:

1. A method for producing a glycoprotein by culturing a genetically recombinant host in a medium, wherein the sugar chain structure binding to a protein is modified by changing the specific sugar consumption rate (consumption rate of sugar in the medium) as an index;
2. The method for producing a glycoprotein according 1, wherein the host is a eukaryote;
3. The method for producing a glycoprotein according to 1, wherein the host is an animal cell, a yeast, or a fungus;
4. The method for producing a glycoprotein according to 1, wherein the host is a CHO cell;
5. The method for producing a glycoprotein according to any one of 1 to 4, wherein the modifying of the sugar chain structure includes at least one selected from:

    1) modifying the amount of total sugar chain;
    2) modifying the ratio of the fucosylated sugar chain in the total sugar chain;
    3) modifying the amount of triantennary type structure sugar chains;
    4) modifying the amount of complete galactose sugar chain;
    5) modifying the amount of natural-type sugar chain; and

6) modifying the kind or molecular weight of sugar chain;

6. The production method according to 1, wherein the modifying of the sugar chain structure includes at least one selected from:

    1) decreasing the ratio of the fucosylated sugar chain in the total sugar chain;
    2) decreasing the amount of triantennary type structure sugar chains;
    3) increasing the amount of complete galactose sugar chain; and
    4) increasing the amount of natural-type sugar chain;

7. The method for producing a glycoprotein according to any one of 1 to 6, wherein the changing of the specific sugar consumption rate as an index is achieved by controlling of the condition concerning the metabolism of sugars;
8. The method for producing a glycoprotein according to 7, wherein the controlling of the condition concerning the sugar metabolism includes controlling at least one selected from:

    1) the concentration of the sugar in the medium;
    2) the composition of the sugar in the medium;
    3) the addition timing of the sugar into the medium; and
    4) the addition of a substance affecting the sugar metabolism in the medium;

9. The method for producing a glycoprotein according to 7 or 8, wherein the changing of the specific sugar consumption rate as an index includes increasing of the specific sugar consumption rate;
10. A method for producing a glycoprotein by culturing a genetically recombinant CHO cell in a medium, wherein the ratio of the fucosylated sugar chain in a sugar chain binding to a protein is decreased by the increasing of the specific sugar consumption rate as an index;
11. A novel glycoprotein obtained by the method for producing a glycoprotein according to any one of 1 to 10;
12. The method for producing a glycoprotein according to 8, wherein the sugar to be added to the medium is glucose, mannose or fructose;
13. The method for producing a glycoprotein according to 8, wherein the concentration of the sugar to be added to the medium is of the extent of 0.75 to 3 g/l;
14. The method for producing a glycoprotein according to 8, wherein the substance affecting the sugar metabolism is an alcohol, retinoic acid, a fatty acid or glutamine;
15. The method for producing a glycoprotein according to 8, wherein the amount of an alcohol to be added is of the extent of 0.03% or less, and the amount of retinoic acid to be added is of the extent of 1 nM to 1 $\mu$M, and the amount of glutamine to be added is of the extent of 0.3 g/l or less; and
16. The method for producing a glycoprotein according to 8, wherein the combination of components to be added to the medium is (glucose and mannose), (glucose and an alcohol), (glucose and retinoic acid), (glucose and glutamine), or (glucose and retinoic acid).

Details will be described hereinbelow.

BEST MODE FOR CARRYING OUT THE INVENTION

The glycoprotein according to the present invention is a generic name for substances having a sugar chain binding to proteins by covalent bonds. It refers to a complex glycoprotein having a sugar chain, which consists of two to six different monosaccharides, which does not have any repeated structure, and which is binding to proteins by covalent bonds.
Such a glycoprotein includes substances that occur widely in the living world, have a biologically and/or physiologically activity, and have sugar chain(s) binding with at least one amino acid residue that can be binding by a sugar chain such as asparagine moiety, serine moiety, and threonine moiety in the amino acid sequence of the protein.
Sugar chains of glycoproteins are classified into two groups based on the binding mode between sugar chain and protein, i.e., serum-type sugar chain and mucin-type sugar chain. The former is an N-linked oligosaccharides sugar chain or asparagines type sugar chain (Asn type sugar chain) obtained by binding, to form an N-$\beta$-glycoside bond, N-acetylglucosamine to Asn of the amino acid sequence Asn-X-Ser/Thr in a polypeptide, is frequently found in serum glycoproteins, and is called 'serum-type sugar chain'. The latter is an O-linked oligosaccharides sugar chain obtained by binding, to form an O-$\alpha$-glycoside bond, N-acetylgalactosamine to Ser or Thr, is frequently found in mucin that is a mucous protein, and is called 'mucin-type sugar chain'.
Each sugar chain can bind to an amino acid in a protein at any bindable site in the amino acid sequence as long as the biological activity and/or physiological activity are/is kept. Therefore, the number of the sugar chain per one molecule of glycoprotein can be one, two, or more.

Sugars constituting the sugar chain include N-acetylglucosamine, N-acetylgalactosamine, D-mannose, D-galactose, L-fucose, and sialic acid as well as, in plants, D-xylose and D-arabinose. These sugars can bind in any arrangement.

Such glycoproteins include plasma protein (e.g., blood coagulation factors such as factor VIII, factor IX, and factor XIII, immunoglobulin, antithrombin III, prothrombin, thrombin, fibrinogen, fibrin, plasminogen, haptoglobin, $\alpha$1-plasmin inhibitor, $\alpha$1-antitrypsin, transferrin, and heparin cofactor II), interferon (IFN), insulin, various growth factors, urinary trypsin inhibitor, plasminogen activators (e.g., urokinase (UK), prourokinase, tissue plasminogen activator (tPA)), colony formation-stimulating factor (CSF), various receptors, various enzymes, erythropoietin (EPO), interleukin (IL), hormone, lymphokine, and cytokine.

A gene encoding the above protein is introduced into an expression vector system to construct the host-vector system for expression according to the present invention. For the host-vector system, a combination consisting of a replicon derived from a species compatible with the host cell and the said host is used in general. The vector has replication point, promoter, control sequence (enhancer), signal sequence, ribosome-binding site, RNA splice sequence, poly-A addition site, and transcription termination sequence (terminator), and, optionally, can has a marker sequence that permits selecting a phenotype in a transformed cell. In addition, for a high production system, a system for amplifying a gene using dihydrofolate reductase (DHFR) gene can also be used.

Any host capable of producing a glycoprotein can be used for the present invention. Such a host includes one derived from a eukaryote (e.g., animal cell, yeast, fungus, plant cell, and insect cell), auxotroph, and antibiotic-sensitive strain.

The animal cell includes CHO cell (e.g., CHO-K1 cell), COS-7 cell, Vero cell, HeLa cell, WI38 cell, BHK cell, MDCK cell, C127 cell, and variants such as dhfr-deficient strain, HGPRT (hypoxanthine guanine phosphoribosyl transferase)-deficient strain, and ouabain-resistant strains derived from those cells.

The yeast includes ones belonging to the genus *Saccharomyces* (e.g., *S. cerevisiae*), the genus *Picbia* (e.g., *P. pastoris*), and the genus *Kluyveromyces*.

The fungus includes one belonging to the genus *Aspergillus*. The plant cell includes ones derived from potato, tobacco, corn, *Oryzae sativa, Brassica campestris*, soybean, tomato, wheat, barley, and rye.

Well-known methods can be used for preparing a host (transformant) capable of producing a glycoprotein using the genetically recombinant technique and for producing a glycoprotein using the host.

The method for preparing a transformant includes a method for introducing a plasmid directly into a host cell and a method for integrating a plasmid into the chromosome. The former method includes the protoplast polyethylene glycol method and the electroporation method. The latter method comprises the steps of making a plasmid contain a partial DNA sequence of a gene present in the host chromosome and introducing the plasmid or linear fragment thereof into the host chromosome by the homologous recombination using the homologous sequence part.

Transformants are cultured by well-known methods. A culture medium depends on the kind of the host. Yeasts may be cultured, for example, with YPD liquid medium (1% yeast extract, 2% Bacto Peptone, and 2% glucose). Animal cells may be cultured, for example, with basal media (e.g., MEM medium, DMEM medium, RPMI medium, and HamF medium), serum media (e.g., FCS-added one, and bovine serum- or human serum-added one), and serum-free media (e.g., insulin-, peptone-, HSA-, and/or transferrin added ones).

DMEM medium is Dulbecco's modified Eagle medium (*In Vitro* vol. 6, p. 89, 1970). RPMI medium includes RPMI1640 medium (*J. Immunol. Methods* vol. 39, p. 285, 1980; *JAMA* vol. 1999, p. 519, 1957). HamF medium includes HamF12 medium (*Proc. Natl. Acad. Sci. USA* vol. 53, p. 288, 1965).

The serum-free medium can contain, for example, insulin, peptone, transferrin, and HSA (human serum albumin) (Japanese Patent Application Laid-Open (*kokai*) No. Hei 4-234982); insulin, peptone, and non-protein iron source (WO98/00521); recombinant insulin, plant peptone and an inorganic iron salt (Japanese Patent 2625302) or recombinant HSA (WO98/06822); and, if necessary, trace metals such as cobalt, molibdenium, iron, manganese, copper, and zinc with being added as a solution of their inorganic or organic salts or complexes (e.g., Japanese Patent Application Laid-Open (*kokai*) No. 2001-120262, WO98/4680, US-5612196).

The culturing is carried out, usually, at 15-43°C (preferably 30-37°C) or so for 10-200 h or so, if necessary, with aeration and/or agitation, by the batch culture technique, the fed-batch culture technique, or the continuous culture technique.

Methods for culturing recombinant host cells are described below. Although culturing vessels such as culturing device using dish, flask, roller bottle, spinner flask, micro-carrier, microcapsule, and/or hollow fiber can be used for culturing a recombinant host cell according to the present invention, other vessels can also be used.

The culturing method includes the successive subculture usually carried out using the above culturing vessel and the continuous culturing method in which old culture liquid is continuously or intermittently withdrawn from the reactor with or without separating cells from the culture liquid and the same volume of fresh medium is supplied with keeping the culture condition for a long time.

The cell density upon culturing the recombinant host cell is, for example, $10^4$-$10^9$ cells/ml or so. The recombinant host cell can also be cultured at a high cell density, for example, at $10^8$ cells/ml or higher for the present invention.

The production of a glycoprotein using the general recombinant host cell according to the present invention can be carried out by the known means as described, for example, in WO91/06649. In case an animal cell is used as a recombinant host cell, methods described, for example, in Japanese Patent Application Laid-Open (*kokai*) No. Sho 61-177987 and Japanese Patent Application Laid-Open (*kokai*) No. Sho 63-146789 can be used.

The present invention is characterized by modifying the structure of sugar chain binding to a protein by changing the specific-sugar-consumption rate as an index upon producing a glycoprotein by culturing a genetically recombinant host in a medium.

"The specific-sugar-consumption rate' is a consumption rate of a sugar in a medium (change in amount of sugar consumption per unit time) per one cell upon culturing a host.

The specific consumption rate in the batch culture technique is defined by Eq.1:

$$v = \frac{1}{Xv} \left( -\frac{dS}{dt} \right) \qquad (1)$$

wherein $v$ is a specific consumption rate, Xv is a cell density of viable cells, S is a concentration of a sugar, and t is a time.

In practical calculation, with making $v$ constant, Eq. 1 can be integrated with respect to time (t) to give Eq.2:

$$S = -v\int Xv\, dt + S_0 \qquad (2)$$

wherein $v$ is a specific consumption rate, Xv is a cell density of viable cells, S is a concentration of a sugar, $S_0$ is the initial concentration of the sugar, and t is a time.

Plotting S of Eq.2 and values corresponding to Eq.3 blow and calculating the slope give a specific consumption rate $v$.

$$\int Xv\, dt \qquad (3)$$

wherein Xv is a cell density of viable cells, and t is a time.

A specific consumption rate in continuous culture is expressed by Eq.4:

$$V\frac{dS}{dt} = F\,(Sin - S) - vVXv \qquad (4)$$

wherein $v$ is a specific consumption rate, Xv is a cell density of viable cells, S is a concentration of a sugar, t is a time, V is a culture volume, F is a flow rate, and Sin is a concentration of the sugar in the influent.

Integration of Eq.4 gives Eq.5:

$$S - \frac{F}{V}\int (Sin - S)dt = -v\int Xv\, dt + S_0 \qquad (5)$$

wherein $v$ is a specific consumption rate, Xv is a cell density of viable cells, S is a concentration of a sugar, So is the initial concentration of the sugar, t is a time, V is a culture volume, F is a flow rate, and Sin is a sugar concentration in the influent.

Plotting values corresponding to the left side of Eq.5 and values corresponding to Eq.6 below and calculating the slope give the specific consumption rate $v$:

$$\int Xv\, dt \qquad (6)$$

wherein Xv is a cell density of viable cells, and t is a time.

Changing the specific consumption rate of a sugar as an index can be carried out by controlling of the condition concerning the metabolism of the sugar, for example, by changing the concentration, the composition, and the addition stage of sugar(s) (e.g., glucose, mannose, and fructose) in a medium with or without adding substance(s) that affect(s) the sugar metabolism (e.g., alcohol, retinoic acid, fatty acid, and glutamine). It is preferable to increase the specific consumption rate of a sugar for the present invention.

The concentration of sugar(s) is, for example, 0.75-3 g/l or so. These constituents can be added in a combination such as (glucose and mannose), (glucose and an alcohol), (glucose and retinoic acid), (glucose and glutamine), and (an alcohol and retinoic acid). Each of these constituents can be simultaneously added as one dose, independently added by several times doses, simultaneously and continuously added, independently and continuously added, or independently and by combination consisting of "continuous dose of one regent" and "one dose or several times doses of another reagent".

Alcohol(s) can be added to be 0.03% or less or so. Retinoic acid can be added to be 1 nM to 1 μM or so. Glutamine can be added to be 0.3 g/l or less or so.

'Modifying the structure of a sugar chain' means 'modifying the total amount of a sugar chain', 'modifying the amount of fucosylated sugar chain', 'modifying the amount of triantennary type structure sugar chains ', 'modifying the amount of complete galactose sugar chain', 'modifying the amount of natural-type sugar chain', and 'modifying the kind and/or molecular weight of sugar chain as the concept'. It is preferable to decrease the amount of fucosylated sugar chain, to decrease the amount of triantennary type structure sugar chains, to increase the amount of complete galactose sugar chain, to increase the amount of natural-type sugar chain, and so on for the present invention.

Cells expressing a glycoprotein according to the present invention via the gene manipulation can be treated by well-known methods such as the freezing and thawing method, the glass bead method, the high-pressure method, the ultrasonication method, and the enzyme method in the case of the intracellular expression to give a crude extract containing a glycoprotein according to the present invention. In the case of the extracellular expression (secretory expression), a glycoprotein according to the present invention can be obtained from the culture supernatant.

The glycoprotein can be purified by well-known methods such as the fractionation, the ultrafiltration, the gel filtration, the ion exchanger treatment, the affinity chromatography, the adsorption chromatography, the centrifugation, the dialysis, and the porous membrane treatment.

A glycoprotein according to the present invention can be pharmaceutically prepared by well-known techniques for the pharmaceutical preparation.

Examples

Although examples and experimental examples are given in order to describe the present invention more in detail, the present invention is not limited to these examples.

Reference Example

A dhfr-deficient strain of CHO-K1 cell into which human antithrombin-III (hereinafter referred to as AT-III) gene and dhfr gene were integrated was adapted to a low-serum medium containing 0.5% fetal bovine serum (FBS) (13D-35D) according to the method disclosed in Japanese Patent Application Laid-Open (*kokai*) No. Hei 4-349880 or *Biosci. Biotech. Biochem.* vol. 56, 600-604, 1992 to provide for examples and experimental examples of the present invention.

Example 1

The cell described in Reference Example was cultured in a flask to a confluent state, and 0.25% trypsin solution was added to the resultant cells, and the obtained mixture was incubated at 37°C for 10 min, and an equivalent volume of a fresh medium was added to the resultant mixture, and pipetting was carried out, and the obtained mixture was centrifuged, and the obtained supernatant was discarded, and a fresh medium was added to the pellet, and cells were suspend, and an appropriate amount of the obtained suspension was inoculated to a fresh vessel for the successive subculture. After the successive subculture was carried out four times, cells were suspended in a fresh medium to give a preculture.

A medium was prepared by adding glucose to 0.5% FBS-containing α-MEM medium to be 1.5 g/l. The composition of the medium is described below in detail:

200 ml of the medium was prepared by mixing 184 ml of α-MEM medium [8.77 g of α-MEM powder (Nissui Pharm. Co.), 2.2 g of NaHCO$_3$, 0.292 g of L-glutamine, an appropriate amount of hydrochloric acid, followed by adding ultra-pure water up to 1L, pH7.2], 1ml of FBS (ICN Co.), 2 ml of a penicillin-streptomycin solution (Gibco Co.), 500 μl of 2M MTX solution [prepared by mixing 50.8 mg of L-(+)-amethopterin (Nakalai Tesque Inc.), 818 mg of NaCl, 559 mg of HEPES, 50 mg of $Na_2HPO_4 \cdot 12H_2O$, an appropriate amount of hydrochloric acid, followed by adding ultra-pure water up to 50 ml, pH7.1], 200 µl of 1 g/l insulin solution, 10 ml of 10% soy peptone solution, 20 µl of 10 g/l $FeSO_4 \cdot 7H_2O$ solution, 66 µl of trace metal solution [prepared by mixing 1.1 mg of $CoCl_2 \cdot 6H_2O$, 2.5 mg of $CuSO_4 \cdot 5H_2O$, 23.8 mg of $FeSO_4 \cdot 7H_2O$, 0.8 mg of $MnSO4 \cdot 4H_2O$, 1.25 mg of $(NH_4)_6Mo_7O_{24} \cdot 4H_2O$, 1.5 mg of $ZnSO_4 \cdot 7H_2O$, followed by adding ultra-pure water up to 20 ml], and 2 ml of 20% glucose.

Cells were inoculated into 10 ml of the medium from the preculture to give an initial cell density of $0.43 \times 10^5$ cells/ml, and batch culture was carried out under the atmosphere of 5% $CO_2$ at 37°C for 113 h to produce recombinant AT-III (rAT-III) in the culture medium.

Example 2

Culture was carried out according to Example 1 except that glucose was added at 3 g/l to produce rAT-III in the culture medium.

Example 3

Culture was carried out according to Example 1 except that glucose was further added to give a final concentration of 1.5 g/l after 41 h from the initiation of the culture to produce rAT-III in the culture medium.

Example 4

Culture was carried out according to Example 1 except that mannose was further added to give a final concentration of 1.5 g/l after 41 h from the initiation of the culture to produce rAT-III in the culture medium.

Experimental Example 1

Relation between concentration/method for addition of sugar and specific sugar consumption rate.

A specific sugar consumption rate was calculated at the logarithmic growth phase for each culture system hypothesizing that the cell density of total cells was $0.45 \times 10^5$ cells/ml and that of viable cells was $0.43 \times 10^5$ cells/ml with these values being the same as those of the inoculum at the initiation of the culture. A sugar consumption rate of the culture system of Example 3 was calculated by adding an assumed value calculated from the assayed sugar concentration before the re-addition and an assumed increase in the sugar concentration by the re-addition. Glucose concentrations were assayed using a Glucose Analyzer (Yellow Spring Inc., Model 127). Culture was carried out three times for each condition. Results are summarized in Table 1.

Table 1

|  | Concentration of glucose (g/l) | Specific sugar consumption rate (x $10^{-10}$ g/cell·hr) |
|---|---|---|
| Example 1 | 1.5 | $1.17 \pm 0.07$ |
| Example 2 | 3 | $1.25 \pm 0.07$ |
| Example 3 | 1.5 + 1.5 | $1.35 \pm 0.10$ |
| Specific sugar consumption velocities in the table are 'average values' $\pm$ 'standard deviations'. | | |

Increase in glucose concentration enhanced the specific sugar consumption rate. Re-addition of glucose in the course of the culture also enhanced the specific sugar consumption rate.

Experimental Example 2

Relation between specific sugar consumption rate and sugar chain structure

Sugar chain structure of rAT-III produced in each culture system was analyzed. Culture supernatant was collected, and rAT-III was purified/collected using an antibody column [CNBr-activated agarose (Sepharose 4B, Pharmacia) fixed with rabbit anti-human AT-III antibody (DAKO, A296)]. Tris buffer (pH7.5) containing 0.5M NaCl and 0.5% Tween 80 was used for the adsorption; Tris buffer (pH7.5) containing 4.5M magnesium chloride for the desorption.
Collected rAT-III was incubated with 0.01N hydrochloric acid at 80°C for 1 h, and the resultant mixture was treated with pepsin at pH2, 37°C for 1 day, and the pepsin was inactivated, and the resultant mixture was treated with Glycopeptidase A (Seikagaku Kogyo Co.) at 37°C overnight to excise the sugar chain, and the excised sugar chain was PA-labelled by incubating with 2-aminopyridine(Wako) at 90°C for 1 h, and the excised PA-labelled origosaccharides was reduced by reacting with boron-dimethylamine complex (Wako Pure Chem. Industries, Ltd.) at 80°C for 35 min, and pH of the obtained reaction solution was adjusted to pH10 with ammonia solution, and chloroform was added to the resultant reaction solution, and the obtained mixture was mixed, and an aqueous fraction was collected.

The aqueous solution was lyophilized, and the obtained solid was dissolved in 10 mM ammonium sulfate (pH 6), and the obtained solution was analyzed by the reversed-phase HPLC using Shimadzu Seisakusho LC10AD liquid chromatograph system, a C18-based reversed-phase column (Cosmosil 5C18-AR30, φ 6 mm x 150 mm, Nacalai Tesque Inc.), flow rate of 2 ml/min, column temperature of 37°C, a detector by fluorescence spectrophotometry, an excitation wavelength at 320 nm, detection wavelength at 400 nm, 20 mM ammonium sulfate buffer (pH 4) as eluate A, and

20 mM ammonium sulfate buffer (pH 4) containing 1% 1-butanol as eluate B. The eluting program was as follows: linear gradient elution from time 0 min to time 10 min, (eluate A: eluate B) was from (100:0) to (90:10); linear gradient elution from time 10 min to time 25 min, (eluate A: eluate B) was from (90:10) to (95:5); isocratic elution from time 25 min, (eluate A: eluate B) was 95:5.

Peaks A-J were detected by the above analysis. The structures of the sugar chains of peaks A, B, C, D, G, H, and J in the chromatograph were estimated based on the retention times of authentic PA-labelled sugar chains and the two-dimensional map. As authentic PA-labelled sugar chain, PA-sugar chain 012 (Takara Co., 4112) for peak A, PA-sugar chain 001 (Takara Co., 4101) for peak D, PA-sugar chain 009 (Takara Co., 4109) for peak H, PA-sugar chain 010 (Takara Co., 4110) for peak J, PA-oligosaccharide (Seikagaku Kogyo Co., Y-C207) for peak B, PA-oligosaccharide (Seikagaku Kogyo Co., Y-C208) for peak C, and PA-oligosaccharide (Seikagaku Kogyo Co., Y-C228) for peak G. The sugar chain structure of E,F,I was estimated based only on the two-dimensional map. The two-dimensional map method was carried out using the combination of normal-phase HPLC and reversed-phase HPLC according to the method disclosed in Japanese Patent Laid-Open Hei 11-127890. Estimated structures of each sugar chain are illustrated in table 2.

Table 2

| PEAK | ESTIMATED STRUCTURES |
|---|---|
| A | GlcNAcβ1−2Man α1 <br> GlcNAcβ1−2Man α1 ⟩ Manβ1−4GlcNAcβ1−4GlcNAc |
| B | Galβ1−4GlcNAcβ1−2Man α1 <br> GlcNAcβ1−2Man α1 ⟩ Manβ1−4GlcNAcβ1−4GlcNAc |
| C | GlcNAcβ1−2Man α1 <br> Galβ1−4GlcNAcβ1−2Man α1 ⟩ Manβ1−4GlcNAcβ1−4GlcNAc |
| D | Galβ1−4GlcNAcβ1−2Man α1 <br> Galβ1−4GlcNAcβ1−2Man α1 ⟩ Manβ1−4GlcNAcβ1−4GlcNAc |
| E | GlcNAcβ1−2Man α1 <br> GlcNAcβ1−2Man α1 ⟩ Manβ1−4GlcNAcβ1−4GlcNAc   (Fucα1→6) |
| F | Galβ1−4GlcNAcβ1−2Man α1 <br> GlcNAcβ1−2Man α1 ⟩ Manβ1−4GlcNAcβ1−4GlcNAc   (Fucα1→6) |
| G | GlcNAcβ1−2Man α1 <br> Galβ1−4GlcNAcβ1−2Man α1 ⟩ Manβ1−4GlcNAcβ1−4GlcNAc   (Fucα1→6) |
| H | Galβ1−4GlcNAcβ1−2Man α1 <br> Galβ1−4GlcNAcβ1−2Man α1 ⟩ Manβ1−4GlcNAcβ1−4GlcNAc   (Fucα1→6) |
| I | Galβ1−4GlcNAcβ1 <br> Galβ1−4GlcNAcβ1 ⟩ Man α1 <br> Galβ1−4GlcNAcβ1−2Man α1 ⟩ Manβ1−4GlcNAcβ1−4GlcNAc   (Fucα1→6) |
| J | Galβ1−4GlcNAcβ1−2Man α1 <br> Galβ1−4GlcNAcβ1 <br> Galβ1−4GlcNAcβ1 ⟩ Man α1 ⟩ Manβ1−4GlcNAcβ1−4GlcNAc   (Fucα1→6) |

The distribution of the sugar chain structure of rAT-III produced in each culture system was determined once. Ratios of areas of peaks A-J of chromatographs obtained were calculated as the ratios of the amounts of sugar chain structures. Similar analyses were carried out also for human AT-III derived from plasma (Neuart[Trade Name], Welfide Corp.) as the control. Results are summarized in Table 3.

Table 3

| | Ratio of each sugar chain per total sugar chain (%) | | | |
|---|---|---|---|---|
| | Example 1 | Example 2 | Example 3 | AT-III derived from plasma |
| A | 0 | 0 | 0 | 4 |
| B | 0 | 0 | 0 | 7 |
| C | 24 | 27 | 30 | 8 |

Table 3   (continued)

|  | Ratio of each sugar chain per total sugar chain (%) | | | |
|---|---|---|---|---|
|  | Example 1 | Example 2 | Example 3 | AT-III derived from plasma |
| D | 15 | 14 | 14 | 81 |
| E | 2 | 3 | 3 | 0 |
| F+G | 7 | 8 | 5 | 0 |
| H | 46 | 43 | 42 | 0 |
| I | 3 | 2 | 3 | 0 |
| J | 4 | 2 | 3 | 0 |

Experimental Example 3

Relation between specific sugar consumption rate and amount of total sugar chain per AT-III

An amount of total sugar chain per AT-III was investigated for each culture expression. An amount of sugar chain binding to fucose per AT-III was assayed three times per one sample by the AAL-binding assay method using biotin-labelled *Aleuric aurantia* lectin (AAL, Wako Pure Chem. Industries, Ltd., 018-14691).

The AAL method is for assaying an amount of sugar chain binding to fucose using a lectin that is a protein that binds sugar-specifically. The antigen-antibody was made occur between an anti-AT-III antibody adsorbed on a solid layer and AT-III, and biotin-labelled lectin (AAL) was made react with the sugar chain of AT-III, and the amount of sugar chain binding to fucose was determined as the concentration of an enzyme by serially adding 100 µl of 18.6 µg/ml anti-AT-III antibody, 100 µl of 10 µg/ml AT-III,100 µl of 40 µg/ml labelled AAL and 100 µl of 2 µg/ml streptavidin-labelled enzyme (alkaline protease). Diethanolamine was used as the substrate for the enzyme.

A relative ratio of the amount of total sugar chain binding to fucose per AT-III in each culture system was calculated by dividing the value obtained by the above method with the ratio of the amount of sugar chain binding to fucose per the amount of total sugar chain (obtained in Example 2). Relative values were calculated with the value of Example 1 being 1, and are summarized in Table 4.

Table 4

|  | amount of total sugar chain per AT-III |
|---|---|
| Example 1 | $1.00 \pm 0.07$ |
| Example 2 | $1.05 \pm 0.04$ |
| Example 3 | $1.15 \pm 0.10$ |
| Values in the table indicate 'averages' $\pm$ 'standard deviations'. | |

As the specific sugar consumption rate increases, the amount of total sugar chain increased.

Experimental Example 4

The relation between the specific sugar consumption rate and the ratio of sugar chain binding to fucose per total sugar chain was investigated. Ratios of sugar chain binding to fucose to the total sugar chain were calculated by calculating the sum of sugar chains binding to fucose (peaks E to J) in results of Example 2 (Table 3), and were summarized in Table 5.

Table 5

|  | Ratio of sugar chain binding to fucose to total sugar chain (%) |
|---|---|
| Example 1 | 61 |
| Example 2 | 59 |
| Example 3 | 56 |

Table 5 (continued)

| | Ratio of sugar chain binding to fucose to total sugar chain (%) |
|---|---|
| AT-III derived from plasma | 0 |

As the specific sugar consumption rate increases, the ratio of sugar chain binding to fucose to the total sugar chain decreased. Sugar chain binding to fucose was not detected with respect to AT-III derived from plasma.

Experimental Example 5

The relation between a specific sugar consumption rate and a sugar chain found in AT-III derived from plasma (natural type) was investigated. Ratios to the amount of total sugar chain were calculated based on the sum of sugar chains (peaks A to D) found in the natural type in the results (Table 3) of Example 2. With respect to the ratio to AT-III, a relative value of each sugar chain amount to AT-III was calculated by amplifying a ratio to the total sugar chain with a value of the total sugar chain amount to AT-III, and was expressed as a relative value with the value of the culture system of Example 1 being 1. Results are summarized in Table 6.

Table 6

| | Ratio of sugar chain found in natural type | |
|---|---|---|
| | Value to total sugar chain (%) | Value per AT-III (relative value) |
| Example 1 | 39 | $1.00 \pm 0.07$ |
| Example 2 | 41 | $1.09 \pm 0.04$ |
| Example 3 | 44 | $1.28 \pm 0.10$ |
| AT-III derived from plasma | 100 | - |

As the specific sugar consumption rate increased, the ratio of sugar chains found in the natural type increased.

INDUSTRIAL APPLICABILITY

The method according to the present invention permits producing a glycoprotein whose sugar chain structure was modified by changing the specific sugar chain consumption rate as an index in the culture of a genetically recombinant host. In a word, it was suggested that the sugar chain modification could be controlled by operating the specific sugar consumption rate.

**Claims**

1. A method for producing a glycoprotein by culturing a genetically recombinant host in a medium, wherein the sugar chain structure binding to a protein is modified by changing the specific sugar consumption rate (consumption rate of sugar in the medium) as an index.

2. The method for producing a glycoprotein according to claim 1, wherein the host is a eukaryote.

3. The method for producing a glycoprotein according to claim 1, wherein the host is an animal cell, a yeast, or a fungus.

4. The method for producing a glycoprotein according to claim 1, wherein the host is a CHO cell.

5. The method for producing a glycoprotein according to any one of claims 1 to 4, wherein the modifying of the sugar chain structure includes at least one selected from:

    1) modifying the amount of total sugar chain;
    2) modifying the ratio of the fucosylated sugar chain in the total sugar chain;
    3) modifying the amount of triantennary type structure sugar chains;
    4) modifying the amount of complete galactose sugar chain;

5) modifying the amount of natural-type sugar chain; and

6) modifying the kind or molecular weight of sugar chain.

**6.** The production method according to claim 1, wherein the modifying of the sugar chain structure includes at least one selected from:

1) decreasing the ratio of the fucosylated sugar chain in the total sugar chain;

2) decreasing the amount of triantennary type structure sugar chains;

3) increasing the amount of complete galactose sugar chain; and

4) increasing the amount of natural-type sugar chain.

**7.** The method for producing a glycoprotein according to any one of claims 1 to 6, wherein the changing of the specific sugar consumption rate as an index is achieved by controlling of the condition concerning the metabolism of sugars.

**8.** The method for producing a glycoprotein according to claim 7, wherein the controlling of the condition concerning the sugar metabolism includes controlling at least one selected from:

1) the concentration of the sugar in the medium;

2) the composition of the sugar in the medium;

3) the addition stage of the sugar into the medium; and

4) the addition of a substance affecting the sugar metabolism in the medium.

**9.** The method for producing a glycoprotein according to claim 7 or 8, wherein the changing of the specific sugar consumption rate as an index includes increasing of the specific sugar consumption rate.

**10.** A method for producing a glycoprotein by culturing a genetically recombinant CHO cell in a medium, wherein the ratio of the fucosylated sugar chain in a sugar chain binding to a protein is decreased by increasing of the specific sugar consumption rate as an index.

**11.** A novel glycoprotein obtained by the method for producing a glycoprotein according to any one of claims 1 to 10.

**12.** The method for producing a glycoprotein according to claim 8, wherein the sugar to be added to the medium is glucose, mannose or fructose.

**13.** The method for producing a glycoprotein according to claim 8, wherein the concentration of the sugar to be added to the medium is of the extent of 0.75 to 3 g/l.

**14.** The method for producing a glycoprotein according to claim 8, wherein the substance affecting the sugar metabolism is an alcohol, retinoic acid, a fatty acid or glutamine.

**15.** The method for producing a glycoprotein according to claim 8, wherein the amount of an alcohol to be added is of the extent of 0.03% or less, and the amount of retinoic acid to be added is of the extent of 1 nM to 1 μM, and the amount of glutamine to be added is of the extent of 0.3 g/l or less.

**16.** The method for producing a glycoprotein according to claim 8, wherein the combination of components to be added to the medium is (glucose and mannose), (glucose and an alcohol), (glucose and retinoic acid), (glucose and glutamine), or (glucose and retinoic acid).

## INTERNATIONAL SEARCH REPORT

| International application No. |
| --- |
| PCT/JP01/05812 |

**A. CLASSIFICATION OF SUBJECT MATTER**
Int.Cl$^7$ C12P21/00, C12N15/10, C07K14/00//(C12P21/00, C12R1:91)

According to International Patent Classification (IPC) or to both national classification and IPC

**B. FIELDS SEARCHED**

Minimum documentation searched (classification system followed by classification symbols)
Int.Cl$^7$ C12P21/00-21/02, C12N15/10, C07K1/00-16/46

Documentation searched other than minimum documentation to the extent that such documents are included in the fields searched

Electronic data base consulted during the international search (name of data base and, where practicable, search terms used)
WPI (DIALOG), BIOSIS (DIALOG)

**C. DOCUMENTS CONSIDERED TO BE RELEVANT**

| Category* | Citation of document, with indication, where appropriate, of the relevant passages | Relevant to claim No. |
| --- | --- | --- |
| X | JP 11-127890 A (Nakano Vinegar Co., Ltd.),<br>18 May, 1999 (18.05.99),<br>(Family: none) | 1-16 |
| X | JP 6-292592 A (Snow Brand Milk Products Co., Ltd.),<br>21 October, 1994 (21.10.94),<br>(Family: none) | 1-16 |
| A | JP 9-84582 A (Kirin Brewery Company, Limited),<br>31 March, 1997 (31.03.97),<br>(Family: none) | 1-16 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>05 October, 2001 (05.10.01) | Date of mailing of the international search report<br>23 October, 2001 (23.10.01) |
| --- | --- |
| Name and mailing address of the ISA/<br>Japanese Patent Office | Authorized officer |
| Facsimile No. | Telephone No. |

Form PCT/ISA/210 (second sheet) (July 1992)